(19)

Europäisches Patentamt

European Patent Office

Office européen des brevets

(11) **EP 4 368 108 A1**

(12) **EUROPEAN PATENT APPLICATION**

(43) Date of publication:
**15.05.2024  Bulletin 2024/20**

(21) Application number: **22206530.2**

(22) Date of filing: **10.11.2022**

(51) International Patent Classification (IPC):
***A61B 5/369*** (2021.01)     ***A61B 5/00*** (2006.01)

(52) Cooperative Patent Classification (CPC):
**A61B 5/369; A61B 5/7221;** A61B 5/318;
A61B 5/389; A61B 5/398

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **IDUN Technologies AG**
**8152 Glattpark (CH)**

(72) Inventors:
• **DU TOIT, Wadda**
**8037 Zürich (CH)**

• **DO, Cao Tri**
**8304 Wallisellen (CH)**
• **GISIN, Severine**
**8152 Glattpark (Opfikon) (CH)**
• **BACHMANN, Simon**
**8152 Glattpark (Opfikon) (CH)**
• **THIELEN, Moritz**
**8152 Glattpark (Opfikon) (CH)**

(74) Representative: **Vossius & Partner**
**Patentanwälte Rechtsanwälte mbB**
**Siebertstraße 3**
**81675 München (DE)**

(54)    **METHODS FOR OBTAINING A QUALITY SCORE**

(57)    The present invention relates to a method for obtaining a quality score for an electrophysiological signal, the method including the following steps: obtaining a plurality of metrics from the electrophysiological signal; obtaining a plurality of standardized metrics of the plurality of metrics based on mean and standard deviation values of a plurality of respective metrics from a first predetermined dataset of electrophysiological signals; obtaining a weighting parameter for each of the plurality of standardized metrics based on an overlap between respective standardized metrics of the first predetermined dataset of electrophysiological signals and a second predetermined dataset of electrophysiological signals, an overlap between respective standardized metrics of the first predetermined dataset of electrophysiological signals and a third predetermined dataset of electrophysiological signals, and an area of the respective standardized metrics of the first predetermined dataset of electrophysiological signals; obtaining a plurality of weighted standardized metrics of the plurality of metrics from the electrophysiological signal based on the standardized metrics and the respective weighting parameter; and obtaining a quality score based on the plurality of weighted standardized metrics of the electrophysiological signal.

FIG. 1

EP 4 368 108 A1

**Description**

The human body is producing electrophysiological signals due to heart (ECG), muscle (EMG), eye (EOG) or brain (EEG) activity. These electric potentials can be measured using ExG recording devices, e.g. in-ear EEG devices using electrodes to measure the respective signals. When developing new biopotential recording devices, which includes materials, firmware, and hardware, a metric is necessary to objectively compare and quantify the performance of the ExG data acquisition. Furthermore, when performing ExG experiments, it is important to be notified when a signal cannot be trusted, or when adjustments need to be made to establish a proper or improved ExG measurement.

Current signal quality quantification methods are limited due to the following reasons. Current quality estimation methods are often too complex to be used in real time and therefore cannot be used to provide valuable feedback to the user in a commercial context. In addition, current quality estimation methods often require prior theoretical knowledge on which features of the ExG signals are indicative of a bad and good quality. This may cause subjective quality assessments and thresholds being set in place. Furthermore, current quality estimation methods cannot be used during normal resting state measurements but require a stimulation to induce an expected signal. Moreover, current quality estimation methods often require an estimation of the noise sources, and these noise estimation methods often require numerous ExG channels to be effective.

The current state-of-the-art solutions for estimating the quality of ExG can be categorized into four methods.

1. The most common method is based on skin-electrode impedance assessment. Impedance is a measure of the frequency dependent on contact resistance between the electrode and the subject's skin. The lower the impedance of ExG electrodes when in contact with the skin, the better the overall signal quality. Nathan F. et al.: "Characterizing contact impedance, signal quality and robustness as a function of the cardinality and arrangement of fingers on dry contact EEG electrodes", 2014, 36th Annual International Conference of the IEEE Engineering in Medicine and Biology Society, Tautan A. M. et al.: "Signal Quality in Dry Electrode EEG and the Relation to Skin-electrode Contact Impedance Magnitude", BIODEVICES2014 -International Conference on Biomedical Electronics and Devices2, and Fiedler P. et al.: "Novel flexible Dry multipin electrodes for EEG: Signal quality and interfacial impedance of Ti and TiN coatings", 2013, 35th Annual International Conference of the IEEE Engineering in Medicine and Biology Society (EMBC), are such examples of methods using impedance to validate the signal quality.

2. The second method is using the identification and analysis of biomarkers. Biomarkers are quantitative measures in ExG signals. Examples for methods using biomarkers indicative of EEG data quality are: Lins Caldas A. S. et al: "Towards Automatic EEG Signal Denoising by Quality Metric Optimization", 2020 International Joint Conference on Neural Networks (IJCNN), wherein the authors used known maximum and minimum thresholds of typical EEG frequency bands (delta (1-3 Hz), theta (4-7 Hz), alpha (8-12 Hz), beta (13-30 Hz), and gamma (30-100 Hz)) as well as the spatial distribution of the EEG across the scalp. Thea Radèntz: "Signal Quality Evaluation of Emerging EEG Devices", Front. Physiol., 14 February 2018, Sec. Medical Physics and Imaging used thresholds based on the Berger effect (https://link.springer.com/article/10.1007/BF01797193) states that the alpha band power is supposed to be smaller with the eyes open than closed. This is also known as 'alpha attenuation' or 'alpha block'. The signal-to-noise ratio of an auditory steady-state response such as defined in the work of Liu X.: "Enhanced Auditory Steady-State Response Using an Optimized Chirp Stimulus-Evoked Paradigm", Sensors (Basel), 2019 Feb 12;19(3):748, doi: 10.3390/s19030748 is also often used to determine how well the EEG is measured.

3. The third method uses the quantification of artifacts (e.g. caused by motion of the subject) or noise (e.g. powerline noise). This also includes the standard method of signal-to-noise ratio (SNR), where a type of noise is quantified and compared to the amount of 'clean' EEG measured. Noise/artifacts which are used interchangeably is a broad term that can be categorize into non-physiological and physiological artifacts. Thea Radèntz: "Signal Quality Evaluation of Emerging EEG Devices", Front. Physiol., 14 February 2018, Sec. Medical Physics and Imaging uses artifact subspace reconstruction (ASR) to quantify the noise and then remove it from the raw contaminated EEG to quantify the pure EEG signal. Urigüen J.A. et al.: "EEG artifact removal-state-of-the-art and guidelines" also discusses that source decomposition methods are often used to quantify the amount of electromyography (EMG), electrooculography (EOG), and electrocardiography (ECG) which is then used as the noise in the signal-to-noise calculations. Barry R. J.: "Characterizing pink and white noise in the human electroencephalogram", J Neural Eng., 2021 Mar 16;18(3), doi: 10.1088/1741-2552/abe399 characterizes non-physiological noise such as white and pink noise which can also then be used as the noise for the SNR.

4. The fourth method is statistically comparing measured ExG signals in terms of statistical measures to gold standard datasets of ExG and artifacts. Fickling, S.D et al: "Towards Automatic EEG Signal Denoising by Quality Metric Optimization", 2020 International Joint Conference on Neural Networks (IJCNN) compared standardized statistical metrics from EEG to a standardized gold standard EEG database.

In view of the prior art, it is an object of the present disclosure to provide improved methods for obtaining a quality score for ExG signals.

This object is achieved by the independent claims. Preferred variations and further developments are defined by the features of the dependent claims.

The present disclosure relates to a method for obtaining a plurality of quality metrics for electrophysiological signals, the method including the following steps: obtaining a plurality of metrics from each of: a first predetermined dataset of electrophysiological signals, a second predetermined dataset of electrophysiological signals, and a third predetermined dataset of electrophysiological signals; obtaining a plurality of standardized metrics of the plurality of metrics of each of the first, second and third predetermined datasets of electrophysiological signals using mean and standard deviation values of the plurality of metrics from the first predetermined dataset of electrophysiological signals; and obtaining a plurality of quality metrics as a reduced set of metrics from the plurality of standardized metrics based on an overlap between a) the respective standardized metrics of the first predetermined dataset of electrophysiological signals and the second predetermined dataset of electrophysiological signals, and b) the respective standardized metrics of the first predetermined dataset of electrophysiological signals and the third predetermined dataset of electrophysiological signals.

Various embodiments may preferably implement the following features:
Preferably, the number of the plurality of metrics is 52, but the present disclosure is not limited to this number. In particular, the number of the plurality of metrics can be any number depending on the requirements of the specific application.

Preferably, the overlap between the respective metrics refers to the overlap between the different curves (standardized distributions) of the respective metrics. In other words, each standardized metric has a certain distribution, wherein the respective overlap relates to the overlap of the respective distributions or the overlap of the area underneath the respective curves (overlap of the respective integrals of the respective curves).

Preferably, the overlap represents an association between the respective metrics and/or an equivalence between the respective metrics and/or a similarity between the respective metrics.

Preferably, the plurality of metrics from each of the first, second and third predetermined datasets of electrophysiological signals are extracted from epochs comprising at least 250 samples, preferably between 250 and 7500 samples.

Preferably, the sampling rate of the first, second and third predetermined datasets is 250 samples per second. That is, with this sampling rate, the plurality of metrics from each of the first, second and third predetermined datasets of electrophysiological signals are extracted from 1 second epochs, preferably between 1 and 30 second epochs and most preferably 5 second epochs.

However, other sampling rates are possible and the present disclosure is not limited to a sampling rate of 250 samples per second.

Preferably, the epochs overlap each other by a predetermined amount, wherein preferably a current evaluated epoch comprises 20% to 100% of new data compared to a previous epoch.

The overlap of epochs is used to have a large buffer while still being able to update the quality score with a low latency. The overlap is in particular preferable for low latency updates. In principle any overlap is possible (even no overlap) as long as two conditions are satisfied: the current epoch consists of between 20% to 100% of new data and the current epoch has more than 250 samples.

Preferably, the first predetermined dataset of electrophysiological signals corresponds to signals obtained by clinical gold standard electrode placement, e.g. 10-20 system for EEG, 12-lead precordial lead placement for ECG and the like.

Preferably, in case of EEG data, the first predetermined dataset of electrophysiological signals corresponds to full scalp data, preferably measured at channel T4, and preferably good quality full scalp data. However, the present disclosure is not limited to full scalp data and any dataset of electrophysiological signals that is known to represent good quality electrophysiological signals may be used, preferably from a gold standard database.

In other words, the first predetermined dataset of electrophysiological signals preferably corresponds to data from a gold standard database, preferably obtained by clinical gold standard electrode placement, and more preferably the first predetermined dataset (20) of electrophysiological signals corresponds to full scalp data measured at channel T4, preferably good quality full scalp data.

Preferably, "good quality" in relation to electrophysiological signals as used herein may be defined by a low impedance of the electrode-skin contact, which is a widely accepted measure of good quality data. Impedance is a measure of the frequency dependent on contact resistance between the electrode and the subject's skin. The higher the impedance of the electrode, the smaller the amplitude of the EEG signal. Preferably, good quality data is defined by an impedance of the electrode-skin contact below 10 k$\Omega$, more preferably below 5 k$\Omega$ and most preferably around 100 $\Omega$.

However, the present disclosure is not limited by the measure impedance to define good quality data. Rather, other metrics could also have preferred ranges and quantify good quality data, e.g. SNR >2, amplitude between x and y in µV, etc.

Preferably, obtaining the plurality of metrics comprises obtaining the plurality of metrics for each of a plurality of epochs and/or a plurality of datasets.

Preferably, the second predetermined dataset of electrophysiological signals correspond to in-ear data, preferably good quality in-ear data, and/or the third predetermined dataset of electrophysiological signals correspond to in-ear data, preferably bad quality in-ear data.

Preferably, as used herein "in-ear data" refers to ExG data acquired from in-ear electrodes.

Preferably, the second predetermined dataset of electrophysiological signals and/or the third predetermined dataset of electrophysiological signals correspond to ECG, EMG, or EOG signals and more preferably to EEG signals.

Preferably, obtaining a plurality of quality metrics as a reduced set of metrics comprises: selecting a number of metrics with the largest overlap between the respective standardized metric of the first predetermined dataset of electrophysiological signals and the second predetermined dataset of electrophysiological signals, and/or selecting a number of metrics with the smallest overlap between the respective standardized metric of the first predetermined dataset of electrophysiological signals and the third predetermined dataset of electrophysiological signals.

Preferably, obtaining a plurality of standardized metrics comprises obtaining a Z-score $Z$ for each of the plurality of metrics, wherein $Z$ is obtained by $Z=(X-\mu)/\sigma$, wherein $X$ is the respective metric, $\mu$ is the mean value of the respective metric of the first predetermined dataset of electrophysiological signals and $\sigma$ is the standard deviation value of the respective metric of the first predetermined dataset of electrophysiological signals.

Preferably, the respective metric $X$ refers to the raw value of the respective metric that is extracted from the current epoch. For example, $X$ may refer to the alpha power from a 5 second epoch or the average signal amplitude of a 1 second epoch or the like.

Any of the features/definitions provided in connection with following method for obtaining a quality score percentage function for electrophysiological signals and the method for obtaining a quality score for an electrophysiological signal apply to the method for obtaining a plurality of quality metrics for electrophysiological signals accordingly.

The present disclosure also relates to a method for obtaining a quality score percentage function for electrophysiological signals, the method including the following steps: obtaining a plurality of metrics from each of: a first predetermined dataset of electrophysiological signals, a second predetermined dataset of electrophysiological signals, and a third predetermined dataset of electrophysiological signals; obtaining a plurality of standardized metrics of the plurality of metrics of each of the first, second and third predetermined datasets of electrophysiological signals using mean and standard deviation values of the plurality of metrics from the first predetermined dataset of electrophysiological signals; obtaining a weighting parameter $W$ for each of the plurality of standardized metrics based on an overlap between respective standardized metrics of the first predetermined dataset of electrophysiological signals and the second predetermined dataset of electrophysiological signals, an overlap between respective standardized metrics of the first predetermined dataset of electrophysiological signals and the third predetermined dataset of electrophysiological signals, and an area of the respective standardized metrics of the first predetermined dataset of electrophysiological signals; obtaining a plurality of weighted standardized metrics of the first predetermined dataset of electrophysiological signals based on the standardized metrics and the respective weighting parameter; and obtaining a quality score percentage function based on the plurality of weighted standardized metrics of the first predetermined dataset of electrophysiological signals.

Various embodiments may preferably implement the following features:

Preferably, the plurality of metrics correspond to a plurality of quality metrics obtained by a method according to the above described method for obtaining a plurality of quality metrics for electrophysiological signals. However, the plurality of quality metrics may also be chosen by a different method or based on prior knowledge of the metrics. That is, the method for obtaining a quality score percentage function for electrophysiological signals is not limited to be used in combination with the above described method for obtaining a plurality of quality metrics for electrophysiological signals.

Preferably, the weighting parameter $W$ is obtained by $W=((A-B)/C)^2$, wherein $A$ is the overlap between the respective standardized metrics of the first predetermined dataset of electrophysiological signals and the second predetermined dataset of electrophysiological signals, $B$ is the overlap between the respective standardized metrics of the first predetermined dataset of electrophysiological signals and the third predetermined dataset of electrophysiological signals, and $C$ is the area of the respective standardized metrics of the first predetermined dataset of electrophysiological signals.

Preferably, obtaining a plurality of standardized metrics comprises obtaining a Z-score $Z$ for each of the plurality of metrics, wherein $Z$ is obtained by $Z=(X-\mu)/\sigma$, wherein X is the respective metric, $\mu$ is the mean value of the respective metric of the first predetermined dataset of electrophysiological signals and $\sigma$ is the standard deviation value of the respective metric of the first predetermined dataset of electrophysiological signals.

It is noted that the creation of the Z-score is a standard method used in statistical tests (https://en.wikipedia.org/wiki/Standard_score), which is well known to the skilled person and a detailed description thereof is thus omitted.

Preferably, the respective metric $X$ refers to the raw value of the respective metric that is extracted from the current epoch. For example, $X$ may refer to the alpha power from a 5 second epoch or the average signal amplitude of a 1 second epoch or the like.

Preferably, the plurality of weighted standardized metrics are obtained by multiplying the plurality of standardized metrics with the respective weighting parameter.

Preferably, obtaining the plurality of metrics comprises obtaining the plurality of metrics for each of a plurality of epochs.

Preferably, obtaining a quality score percentage function comprises summing the weighted standardized metrics for each of the plurality of epochs.

Preferably, obtaining a quality score percentage function comprises designating predetermined percentages to each of the plurality of summed weighted standardized metrics, wherein preferably the lowest summed weighted standardized metric is designated 100% and the highest summed weighted standardized metric is designated 50%.

Preferably, obtaining a quality score percentage function comprises fitting the designated predetermined percentages to each of the plurality of summed plurality of weighted standardized metrics, wherein the fitted function corresponds to the quality score percentage function.

Any of the features/definitions provided in connection with the method for obtaining a plurality of quality metrics for electrophysiological signals apply to the method for obtaining a quality score percentage function for electrophysiological signals accordingly.

The present disclosure also relates to a method for obtaining a quality score for an electrophysiological signal, the method including the following steps: obtaining a plurality of metrics from the electrophysiological signal; obtaining a plurality of standardized metrics of the plurality of metrics based on mean and standard deviation values of a plurality of respective metrics from a first predetermined dataset of electrophysiological signals; obtaining a weighting parameter for each of the plurality of standardized metrics based on an overlap between respective standardized metrics of the first predetermined dataset of electrophysiological signals and a second predetermined dataset of electrophysiological signals, an overlap between respective standardized metrics of the first predetermined dataset of electrophysiological signals and a third predetermined dataset of electrophysiological signals, and an area of the respective standardized metrics of the first predetermined dataset of electrophysiological signals; obtaining a plurality of weighted standardized metrics of the plurality of metrics from the electrophysiological signal based on the standardized metrics and the respective weighting parameter; and obtaining a quality score based on the plurality of weighted standardized metrics of the electrophysiological signal.

Various embodiments may preferably implement the following features:
Preferably, the plurality of metrics correspond to a plurality of quality metrics obtained by a method according to the above described method for obtaining a plurality of quality metrics for electrophysiological signals.

Preferably, obtaining a quality score comprises applying a quality score percentage function, preferably obtained by the method for obtaining a quality score percentage function for electrophysiological signals as described above, to the plurality of weighted standardized metrics of the electrophysiological signal.

Preferably, obtaining a quality score comprises obtaining an average of the plurality of weighted standardized metrics of the electrophysiological signal.

Preferably, in an embodiment, obtaining a quality score comprises obtaining a plurality of weighted standardized metrics of the electrophysiological signal for each of a plurality of epochs.

Preferably, obtaining a quality score comprises obtaining a sum of the plurality of weighted standardized metrics of the electrophysiological signal for each of the plurality of epochs.

Preferably, the method comprises obtaining a heatmap based on the plurality of weighted standardized metrics of the electrophysiological signal for each of a plurality of epochs and/or the sum of the plurality of weighted standardized metrics of the electrophysiological signal for each of the plurality of epochs.

Preferably, obtaining a quality score comprises obtaining an average of the sum of the plurality of weighted standardized metrics for each of the plurality of epochs.

Preferably, obtaining a quality score comprises obtaining an average of the sum of the quality scores for each of the plurality of epochs

Any of the features/definitions provided above in connection with the method for obtaining a plurality of quality metrics for electrophysiological signals and/or the method for obtaining a quality score percentage function for electrophysiological signals apply to the method for obtaining a quality score for an electrophysiological signal.

The present disclosure also relates to a combination of the method for obtaining a plurality of quality metrics for electrophysiological signals and/or the method for obtaining a quality score percentage function for electrophysiological signals and/or the method for obtaining a quality score for an electrophysiological signal.

The present disclosure also relates to a computer-implemented method comprising the steps of the method for obtaining a plurality of quality metrics for electrophysiological signals and/or the method for obtaining a quality score percentage function for electrophysiological signals and/or the method for obtaining a quality score for an electrophysiological signal.

The present disclosure also relates to a computer program product comprising instructions which, when the program is executed by a computer, cause the computer to carry out the steps of the method for obtaining a plurality of quality metrics for electrophysiological signals and/or the method for obtaining a quality score percentage function for electrophysiological signals and/or the method for obtaining a quality score for an electrophysiological signal.

In the present disclosure, the use of "obtaining" may encompass "calculating" or "processing", e.g. by the respective unit, component or processor, and "receiving", e.g. from a different unit, component or processor.

The methods disclosed above are thus simple, data driven and can be used in real-time. The methods do not require prior knowledge about which features are indicative of a good ExG signal and require no knowledge on which thresholds to set. The methods are based on resting state data and do not require a stimulation or expected results from the stimulation. The methods do not require any estimations of noise and are thus suitable for single channel ExG signals. Herein, EEG is chosen as an example of a specific use case for quality assessment, but this methodology can be used on any ExG signal where a gold standard database, i.e. predetermined good data, is available.

In particular, in view of Fickling, S.D. et al. mentioned above, the goals and algorithms of the present disclosure are fundamentally different. In particular, Fickling, S.D. et al.'s similar component is used for automatic noise rejection by classifying good and bad short time frames in the time-series signal (called 'epochs'). The goal of the present disclosure's use of a similar component is to evaluate and estimate a quality score for each epoch and entire datasets, which can be used to quantify the overall quality of the signal measurements. This is to aid development of ExG signal measurement devices as well as aid in the analysis of ExG data in terms of measurement capabilities.

In addition, Fickling, S.D. et al. use metrics based on prior knowledge. The methods of the present disclosure provide a pipeline, which eliminates the need for prior knowledge about which metrics to use to evaluate an ExG signal. This makes it possible to create a quality score for any ExG signal where a gold standard database is available. Moreover, Fickling, S.D. et al. weigh all the metrics with the same importance. According to the present disclosure the importance of each metric is quantified and incorporated into the quality score. These weights are based on how similar good ExG signals are from the gold standard database and how different bad ExG signals are from the gold standard database. Fickling, S.D. et al. uses the standard difference to indicate bad and good epochs, whereas the present disclosure adds weighting to the calculation and uses a novel methodology for evaluating and comparing ExG signals to a gold standard database to inform successful or unsuccessful development.

Furthermore, Fickling, S.D. et al. uses a quality indication by summing the standard differences of theoretically informed metrics and using the amount of total zero standard differences as an indication of bad data. In contrast, the present disclosure uses a pipeline that automatically, data driven, identifies the best metrics to use and weighs these metrics according to importance and only then sums the weighted standard differences. In addition, the present disclosure defines quality score percentages according to the distribution of the summed weighted differences. This allows to create a function that converted weighted summed differences to a quality score between 0 and a 100%

The exemplary embodiments disclosed herein are directed to providing features that will become readily apparent by reference to the following description when taken in conjunction with the accompany drawings. In accordance with various embodiments, exemplary systems, methods, devices and computer program products are disclosed herein. It is understood, however, that these embodiments are presented by way of example and not limitation, and it will be apparent to those of ordinary skill in the art who read the present disclosure that various modifications to the disclosed embodiments can be made while remaining within the scope of the present disclosure.

Thus, the present disclosure is not limited to the exemplary embodiments and applications described and illustrated herein. Additionally, the specific order and/or hierarchy of steps in the methods disclosed herein are merely exemplary approaches. Based upon design preferences, the specific order or hierarchy of steps of the disclosed methods or processes can be re-arranged while remaining within the scope of the present disclosure. Thus, those of ordinary skill in the art will understand that the methods and techniques disclosed herein present various steps or acts in a sample order, and the present disclosure is not limited to the specific order or hierarchy presented unless expressly stated otherwise.

Preferred embodiments of the present invention are further elucidated below with reference to the figures. The described embodiments do not limit the present invention.

Fig. 1    shows, in a schematic view, a method for obtaining a plurality of quality metrics for electrophysiological signals according to an embodiment of the present disclosure;

Fig. 2    shows a score comparison between good quality full scalp data, good quality in-ear EEG data and bad quality in-ear EEG data according to an embodiment of the present disclosure;

Fig. 3    shows, in a schematic view, a method for obtaining a quality score for an electrophysiological signal according to an embodiment of the present disclosure;

Fig. 4    shows a comparison of summed weighted Z-scores between good quality full scalp data and bad quality in-ear EEG data according to an embodiment of the present disclosure;

Fig. 5    shows a percentage to quality index conversion function according to an embodiment of the present disclosure;

FIG. 6    shows a flow chart of a method for obtaining a plurality of quality metrics for electrophysiological signals according to an embodiment of the present disclosure;

FIG. 7    shows a flow chart of a method for obtaining a quality score percentage function for electrophysiological signals according to an embodiment of the present disclosure;

FIG. 8    shows a flow chart of a method for obtaining a quality score for an electrophysiological signal according to an embodiment of the present disclosure.

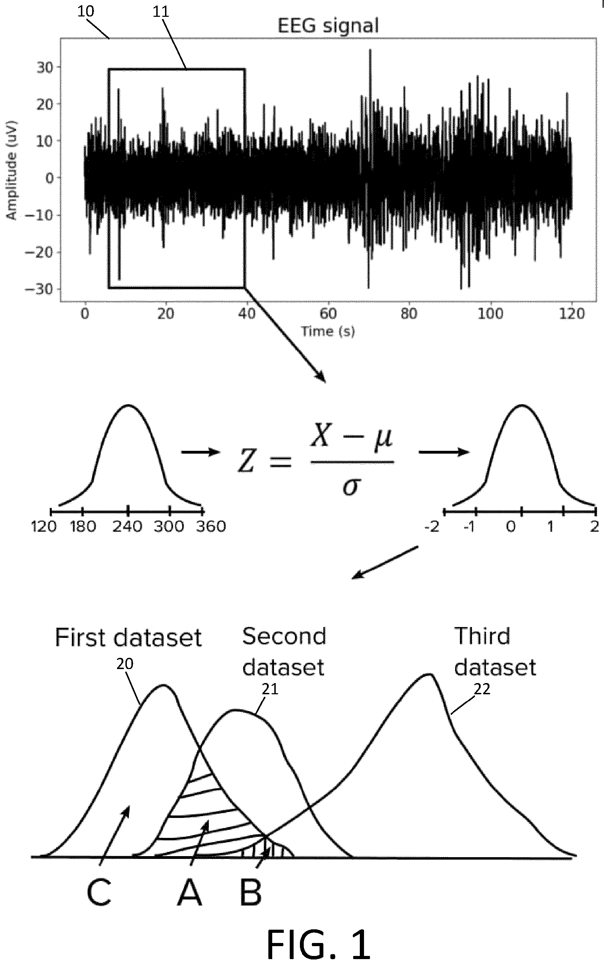

With reference to Fig. 1 a method for obtaining a plurality of quality metrics for electrophysiological signals according to an embodiment of the present disclosure is described. Fig. 1 shows an exemplary EEG signal 10. Fig. 1 also illustrates the use of the Z-score Z to standardize the metrics. Finally, Fig. 1 illustrates the overlap of the different standardized metrics for identifying suitable quality metrics.

In more detail, a plurality of metrics, e.g. 52 metrics, are extracted from 5 second epochs 11 for each dataset (i.e. first dataset 20, second dataset 21 and third dataset 22). In Fig. 1 the black square in the exemplary EEG signal 10 illustrates a 5 second epoch 11. The 5 second epochs 11 overlap each other by 4 seconds. However, the present disclosure is not limited to the specific values for the size of the epochs 11 and the overlap.

Rather, the size of the epochs 11 and the overlap are preferably chosen according to the sample rate. In case the EEG signal 10 has a sample rate of 250 samples per second, the exemplary 5 second epoch 11 would contain 1250 samples. In principle, anything above 250 samples may be sufficient for the method as disclosed herein.

The purpose of the overlap is to have a large buffer while still being able to update the quality score with a low latency. The overlap is thus only necessary for low latency updates. Therefore, 0% overlap is also possible, as long as the following epoch is longer than 250 samples. Therefore, any overlap is possible as long as two conditions are satisfied: the current evaluated epoch consists of between 20% to 100% of new data and the current epoch has > 250 samples.

The good quality full scalp data (first dataset 20) are preferably taken from a gold standard database. The data may be quantified as good data by reference to the impedance of the electrode-skin contact, which is a widely accepted measure of good quality data. In particular, the higher the impedance of the electrode-skin contact, the smaller the amplitude of the EEG signal 10 may be. For example, impedance smaller than 200 kOhm may be acceptable, below 20 kOhm may be good, and below 10 kOhm may be preferable. But other metrics could also have preferred ranges, e.g. SNR >2, amplitude between x and y in $\mu$V, etc.

As can be seen in Fig. 1, the Z-scores Z of the metrics are calculated for each epoch 11 and each dataset using the mean and standard deviation values of the good full scalp data 20 for standardization.

Next, good quality in-ear EEG data (second dataset 21) metrics are standardized using the mean and standard deviation of the respective metrics of the good quality full scalp data 20. In addition, bad quality EEG data (second dataset 22) metrics are standardized using the mean and standard deviation of the respective metrics of the good quality full scalp data 20.

The 52 metrics are then filtered down to only relevant metrics using the following method.

The respective metrics (distributions thereof) of good quality full scalp data 20, good quality in-ear EEG data 21 and bad quality in-ear EEG data 22 are illustrated at the bottom of Fig. 1. The area below the curve of the good quality full scalp data 20 is indicated by C. The overlap between the area below the curve of the good quality full scalp data 20 and the good quality in-ear EEG data 21 is indicated by A and the overlap between the area below the curve of the good quality full scalp data 20 and the bad quality in-ear EEG data 22 is indicated by B.

Two main assumptions are used to filter the metrics down to the most relevant ones: 1) the larger A is, the better the good quality full scalp data 20 and the good quality in-ear EEG data 21 are comparable for this metric, and 2) the smaller B is, the better the metric differentiates good from bad data. Thus, A should preferably be maximized, and B should preferably be minimized. With these assumptions, the most relevant metrics can be identified.

By dividing both A and B by the total area of the good quality full scalp data C, a weighting factor $W$ is calculated as $W=((A-B)/C)^2$. The Z-scores of each metric is calculated as $Z=(X-\mu)/\sigma$, wherein $X$ is the respective metric, $\mu$ is the mean value of the respective metric of the good quality full scalp data 20 and $\sigma$ is the standard deviation value of the respective metric of the good quality full scalp data 21. Thus, a weighted Z-score is obtained as $Z \cdot W$.

The respective weighted Z-scores are then calculated only for the most relevant metrics (according to the above assumptions) for good quality full scalp data 20, good quality in-ear EEG data 21, and bad quality in-ear EEG data 22 for all epochs 11. The weighted Z-scores are summed for each epoch. It should be noted that this step is mainly for evaluation purposes. That is, this step of calculating the respective weighted Z-scores for good quality full scalp data 20, good quality in-ear EEG data 21, and bad quality in-ear EEG data 22 for all epochs 11 is mainly to show that the method of identifying the most relevant metrics performs well. In a real scenario of obtaining a quality score for a measured signal of unknown quality, the identification of the relevant metrics and the calculation of the weighting parameter is not performed with the measured signal, but performed prior to evaluating the measured signal (as outlined in more detail below).

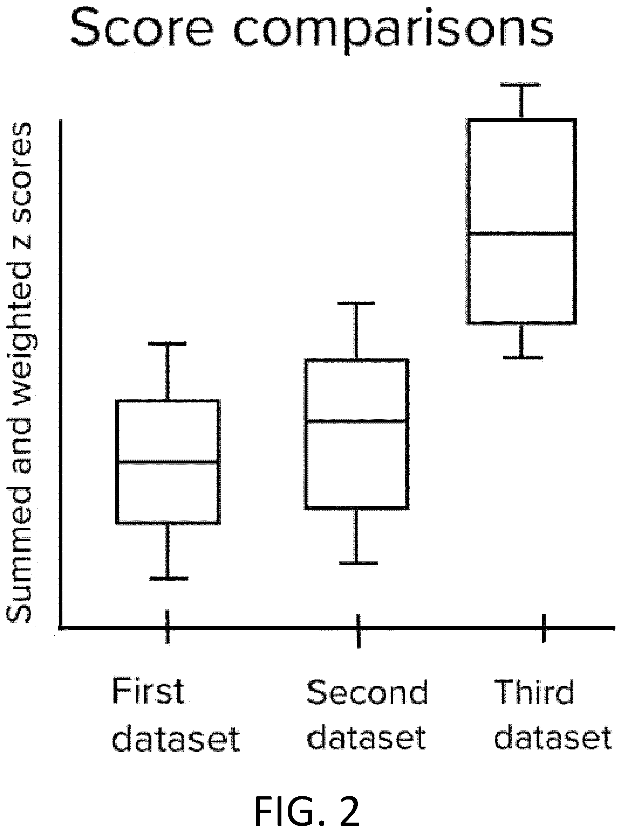

For the present evaluation purposes of whether the method performs well in identifying the most relevant metrics, the plotted result of the summed and weighted Z-scores are shown in Fig. 2. In particular, Fig. 2 shows the summed and weighted Z-scores for the good quality full scalp data 20, the good quality in-ear EEG data 21 and the bad quality in-ear EEG data 22. As can be seen from Fig. 2, the metrics chosen provide a good correlation between good quality full scalp data 20 and good quality in-ear EEG data 21 and also differentiates good from bad data 22.

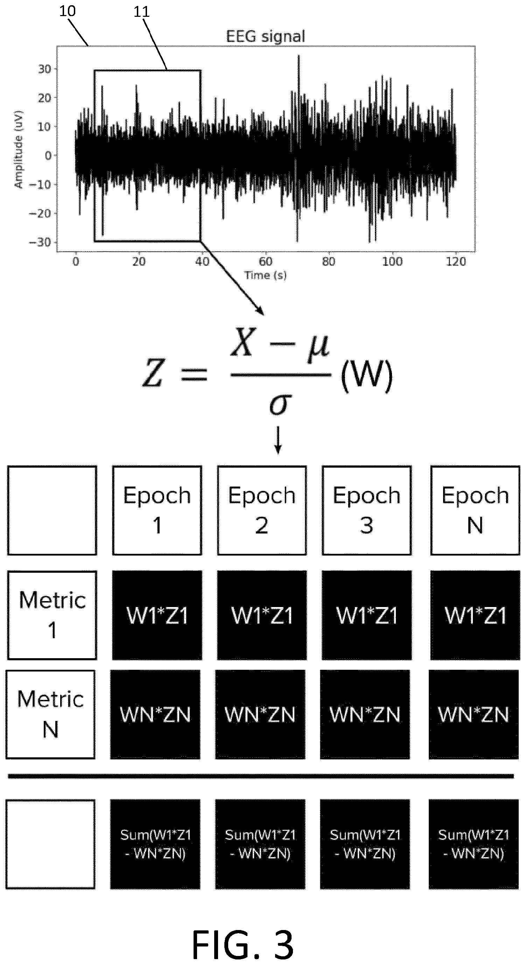

With reference to Figs. 3-5, a method for obtaining a quality score for an electrophysiological signal according to an embodiment of the present disclosure is described.

Fig. 3 shows an exemplary EEG signal 10, wherein an epoch 11 is again indicated by a black square. The weighted Z-scores of the relevant metrics chosen (e.g. chosen by a method as described above) are calculated as described with reference to Figs. 1 and 2 for each epoch 11 and each metric for the electrophysiological signal 10, i.e. the measured in-ear EEG signal of unknown quality using the mean and standard deviation values of the good quality full scalp data 20. That is, the relevant metrics for in-ear EEG signals are determined in accordance with the above described method, i.e. with reference to good quality full scalp data 20, good quality in-ear EEG data 21 and bad quality in-ear EEG data 22 (all of which are pre-characterized as such). The respective metrics are then calculated from the measured in-ear EEG signal of unknown quality using the mean and standard deviation values of the good quality full scalp data 20.

In addition, the weighting parameters are determined as described above for the determined relevant metrics from the, i.e. with reference to good quality full scalp data 20, good quality in-ear EEG data 21 and bad quality in-ear EEG data 22, and multiplied with the Z-scores of the measured in-ear EEG signal of unknown quality to obtain the weighted Z-scores of the measured in-ear EEG signal of unknown quality.

The respective Z-scores can be arranged in a matrix structure as illustrated in Fig. 3. That is, each row may be assigned to a specific metric 1 to N and each column may be assigned to a specific epoch 1 to N. The last row of the matrix may represent the summed weighted Z-scores of each column.

The matrix representation of Fig. 3 may also be visualized by a heatmap, i.e. a color-coded matrix for easy interpretation of the signal under investigation.

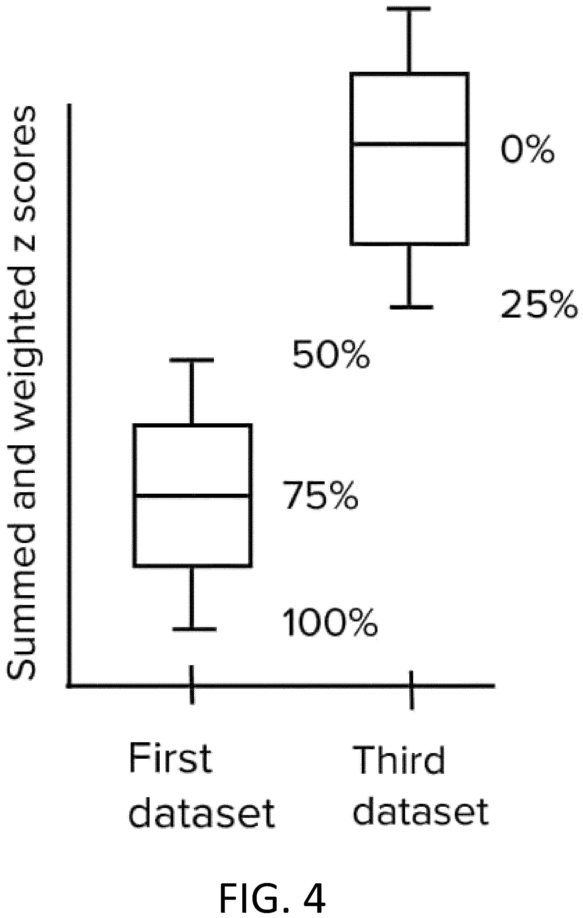

As illustrated in Fig. 4, the summed and weighted Z-scores may be matched with percentages based on the experience of what is considered a good and what is considered a bad Z-score. For example, the lowest summed weighted standardized metric is designated 100% and the highest summed weighted standardized metric is designated 50%. The outliers above that completed the range between 50% and 0%.

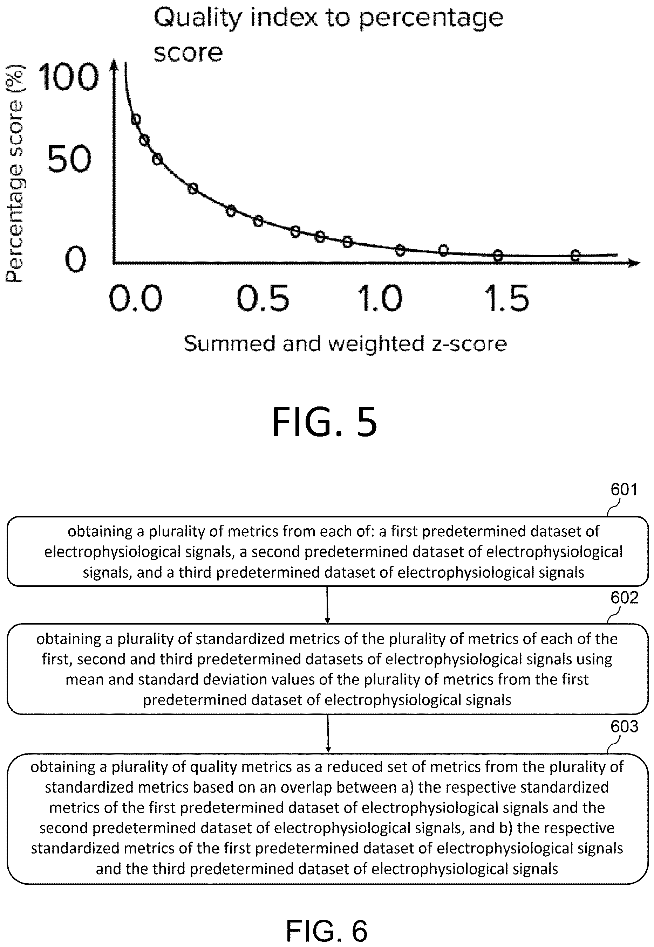

The respective Z-scores with their corresponding x and y values are plotted and a conversion function is fitted through it. The Z-scores are then multiplied by the function shown in Fig. 5 to obtain a percentage value for each epoch. From this, a final percentage score based on the average percentage for all the epoch scores may be obtained as a quality score of the overall signal.

With reference to Figs. 1 to 5, the present disclosure can be summarized as follows.

The first part of the present disclosure is finding the optimal metrics that differentiates good ExG data from bad ExG data. These metrics should be in comparable ranges between good full-scalp EEG data and good in-ear EEG data. 52 metrics were extracted from good full-scalp EEG data at the location T4. T4 is a standard EEG electrode location from the 10-20 system of electrode placement and is in close proximity to the right ear. Therefore, the signals compare to in-ear EEG signals.

The standard Z-scores are calculated for good and bad in-ear EEG by using the gold-standard T4 data as a reference. This means the mean and standard deviation used in the standardisation is always based on the gold-standard database. Standardisation in this context means that the metrics from the in-ear EEG are recalculated by calculating their corresponding Z-score values as shown in the formula for the standard score. In other words, the mean and standard deviation of the good full scalp metrics is used to standardize the good quality in-ear EEG data metrics and the bad quality in-ear EEG metrics.

The metrics that were the most similar in terms of distribution between the standardized full-scalp data and standardized good in-ear data and most different between standardized full-scalp data and the standardized bad in-ear EEG data is used as the final quality metrics (based on the overlap of the curves). That is, the final quality metrics are used as a subset of the 52 metrics.

The second part of the present disclosure is to create a score from the quality metrics. Weighted Z-score values are calculated for the full-scalp data. The lowest weighted Z-score values from the full scalp data is used as a quality score of 100%. The highest weighted Z-score values is used as 50%. The outliers above that completed the range between 50% and 0%. From the full scalp weighted Z-score values the percentages is used as the y-axis and the weighted Z-score values are used as the x-axis and a conversion from Z-score value to quality score percentage function was created.

The third part is the application of the above mentioned methods to obtain a quality score for EEG data. Here, a 5 second epoch is extracted from the time-domain data. The relevant metrics are calculated. The metrics are standardized using the mean and standard deviation from the good full scalp data. The metrics are then multiplied by a weight based on how well the metrics are at differentiating good from bad data as well as how similar are the good in-ear EEG data and the full scalp data for that metric. All the weighted Z-score metrics are then summed to have a final epoch value. This value is then converted to a quality percentage score using the fitted function. This method is then repeated for each overlapping epoch of the dataset and finally all the scores of each epoch is averaged to obtain a score of the complete dataset. The method may be used as real-time quality assessment, i.e. the method may also be applied to only one epoch and there is no need to analyse the final result over all epochs.

Fig. 6 shows a schematic diagram of a method according to an embodiment of the present disclosure. The method may be used for obtaining a plurality of quality metrics for electrophysiological signals. The method includes the following steps:

S601: obtaining a plurality of metrics from each of: a first predetermined dataset of electrophysiological signals, a second predetermined dataset of electrophysiological signals, and a third predetermined dataset of electrophysiological signals;

S602: obtaining a plurality of standardized metrics of the plurality of metrics of each of the first, second and third predetermined datasets of electrophysiological signals using mean and standard deviation values of the plurality of metrics from the first predetermined dataset of electrophysiological signals; and

S603: obtaining a plurality of quality metrics as a reduced set of metrics from the plurality of standardized metrics based on an overlap between a) the respective standardized metrics of the first predetermined dataset of electrophysiological signals and the second predetermined dataset of electrophysiological signals, and b) the respective standardized metrics of the first predetermined dataset of electrophysiological signals and the third predetermined dataset of electrophysiological signals.

In an embodiment, the plurality of metrics from each of the first, second and third predetermined datasets of electrophysiological signals are extracted from epochs comprising at least 250 samples, preferably between 250 and 7500 samples.

In an embodiment, the epochs overlap each other by a predetermined amount, wherein preferably a current evaluated epoch comprises 20% to 100% of new data compared to a previous epoch.

In an embodiment, the first predetermined dataset of electrophysiological signals corresponds to signals obtained by clinical gold standard electrode placement, e.g. 10-20 system for EEG, 12-lead precordial lead placement for ECG and the like.

In an embodiment, in case of EEG, the first predetermined dataset of electrophysiological signals corresponds to full scalp data measured at channel T4, preferably good quality full scalp data.

In other words, in an embodiment, the first predetermined dataset of electrophysiological signals preferably corresponds to data from a gold standard database, preferably obtained by clinical gold standard electrode placement, and more preferably the first predetermined dataset (20) of electrophysiological signals corresponds to full scalp data measured at channel T4, preferably good quality full scalp data

In an embodiment, obtaining the plurality of metrics comprises obtaining the plurality of metrics for each of a plurality of epochs.

In an embodiment, the second predetermined dataset of electrophysiological signals correspond to in-ear data, preferably good quality in-ear data, and/or wherein the third predetermined dataset of electrophysiological signals correspond to in-ear data, preferably bad quality in-ear data.

In an embodiment, obtaining a plurality of quality metrics as a reduced set of metrics comprises: selecting a number of metrics with the largest overlap between the respective standardized metric of the first predetermined dataset of electrophysiological signals and the second predetermined dataset of electrophysiological signals, and/or selecting a number of metrics with the smallest overlap between the respective standardized metric of the first predetermined dataset of electrophysiological signals and the third predetermined dataset of electrophysiological signals.

In an embodiment, obtaining a plurality of standardized metrics comprises obtaining a Z-score $Z$ for each of the plurality of metrics, wherein $Z$ is obtained by

$$Z = \frac{X - \mu}{\sigma}$$

, wherein $X$ is the respective metric, $\mu$ is the mean value of the respective metric of the first predetermined dataset of electrophysiological signals and $\sigma$ is the standard deviation value of the respective metric of the first predetermined dataset of electrophysiological signals.

In an embodiment, the respective metric $X$ refers to the raw value of the respective metric that is extracted from the current epoch. For example, $X$ may refer to the alpha power from a 5 second epoch or the average signal amplitude of a 1 second epoch or the like.

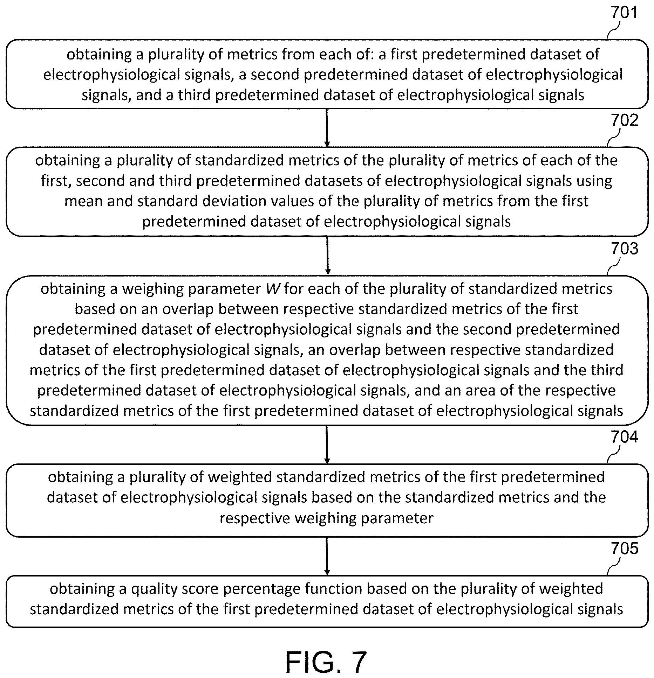

Fig. 7 shows a schematic diagram of a method according to an embodiment of the present disclosure. The method may be used for obtaining a quality score percentage function for electrophysiological signals. The method includes the following steps:

S701: obtaining a plurality of metrics from each of: a first predetermined dataset of electrophysiological signals, a second predetermined dataset of electrophysiological signals, and a third predetermined dataset of electrophysiological signals;

S702: obtaining a plurality of standardized metrics of the plurality of metrics of each of the first, second and third predetermined datasets of electrophysiological signals using mean and standard deviation values of the plurality of metrics from the first predetermined dataset of electrophysiological signals;

S703: obtaining a weighting parameter W for each of the plurality of standardized metrics based on an overlap between respective standardized metrics of the first predetermined dataset of electrophysiological signals and the second predetermined dataset of electrophysiological signals, an overlap between respective standardized metrics of the first predetermined dataset of electrophysiological signals and the third predetermined dataset of electrophysiological signals, and an area of the respective standardized metrics of the first predetermined dataset of electrophysiological signals;

S704: obtaining a plurality of weighted standardized metrics of the first predetermined dataset of electrophysiological signals based on the standardized metrics and the respective weighting parameter; and

S705: obtaining a quality score percentage function based on the plurality of weighted standardized metrics of the first predetermined dataset of electrophysiological signals.

In an embodiment, the plurality of metrics correspond to a plurality of quality metrics obtained by the method for obtaining a plurality of quality metrics for electrophysiological signals as described above.

In an embodiment, the weighting parameter $W$ is obtained by $W = \left(\frac{A-B}{C}\right)^2$, wherein $A$ is the overlap between the respective standardized metrics of the first predetermined dataset of electrophysiological signals and the second predetermined dataset of electrophysiological signals, $B$ is the overlap between the respective standardized metrics of the first predetermined dataset of electrophysiological signals and the third predetermined dataset of electrophysiological signals, and $C$ is the area of the respective standardized metrics of the first predetermined dataset of electrophysiological signals.

In an embodiment, obtaining a plurality of standardized metrics comprises obtaining a Z-score $Z$ for each of the plurality of metrics, wherein $Z$ is obtained by

$$Z = \frac{X-\mu}{\sigma}$$

, wherein $X$ is the respective metric, $\mu$ is the mean value of the respective metric of the first predetermined dataset of electrophysiological signals and $\sigma$ is the standard deviation value of the respective metric of the first predetermined dataset of electrophysiological signals.

In an embodiment, the respective metric $X$ refers to the raw value of the respective metric that is extracted from the current epoch. For example, $X$ may refer to the alpha power from a 5 second epoch or the average signal amplitude of a 1 second epoch or the like.

In an embodiment, the plurality of weighted standardized metrics are obtained by multiplying the plurality of standardized metrics with the respective weighting parameter.

In an embodiment, obtaining the plurality of metrics comprises obtaining the plurality of metrics for each of a plurality of epochs.

In an embodiment, obtaining a quality score percentage function comprises summing the weighted standardized metrics for each of the plurality of epochs.

In an embodiment, obtaining a quality score percentage function comprises designating predetermined percentages to each of the plurality of summed weighted standardized metrics, wherein preferably the lowest summed weighted standardized metric is designated 100% and the highest summed weighted standardized metric is designated 50%.

In an embodiment, obtaining a quality score percentage function comprises fitting the designated predetermined percentages to each of the plurality of summed plurality of weighted standardized metrics, wherein the fitted function corresponds to the quality score percentage function.

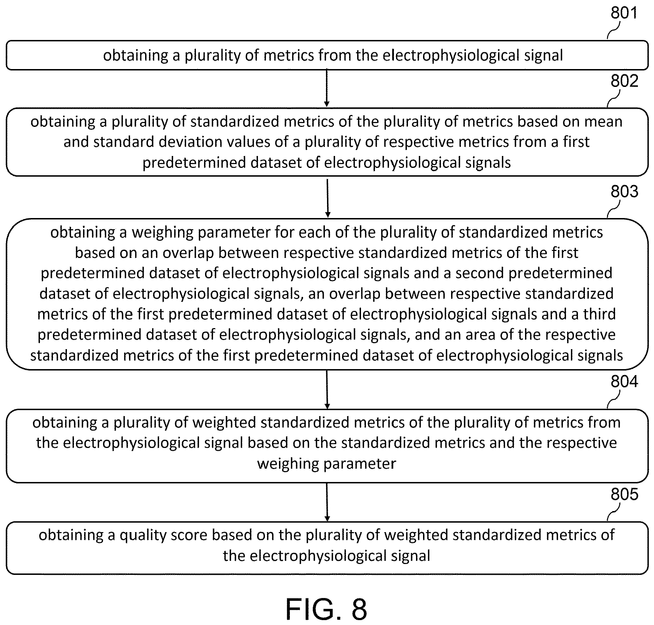

Fig. 8 shows a schematic diagram of a method according to an embodiment of the present disclosure. The method may be used for obtaining a quality score for an electrophysiological signal. The method includes the following steps:

S801: obtaining a plurality of metrics from the electrophysiological signal;

S802: obtaining a plurality of standardized metrics of the plurality of metrics based on mean and standard deviation values of a plurality of respective metrics from a first predetermined dataset of electrophysiological signals;

S803: obtaining a weighting parameter for each of the plurality of standardized metrics based on an overlap between respective standardized metrics of the first predetermined dataset of electrophysiological signals and a second predetermined dataset of electrophysiological signals, an overlap between respective standardized metrics of the first predetermined dataset of electrophysiological signals and a third predetermined dataset of electrophysiological signals, and an area of the respective standardized metrics of the first predetermined dataset of electrophysiological signals;

S804: obtaining a plurality of weighted standardized metrics of the plurality of metrics from the electrophysiological signal based on the standardized metrics and the respective weighting parameter; and

S805: obtaining a quality score based on the plurality of weighted standardized metrics of the electrophysiological signal.

In an embodiment, the plurality of metrics correspond to a plurality of quality metrics obtained by the method for obtaining a plurality of quality metrics for electrophysiological signals as described above.

In an embodiment, obtaining a quality score comprises applying a quality score percentage function, preferably obtained by the method for obtaining a quality score percentage function for electrophysiological signals as described above, to the plurality of weighted standardized metrics of the electrophysiological signal.

In an embodiment, obtaining a quality score comprises obtaining an average of the plurality of weighted standardized metrics of the electrophysiological signal.

In an embodiment, obtaining a quality score comprises obtaining a plurality of weighted standardized metrics of the electrophysiological signal for each of a plurality of epochs.

In an embodiment, obtaining a quality score comprises obtaining a sum of the plurality of weighted standardized metrics of the electrophysiological signal for each of the plurality of epochs.

In an embodiment, the method comprises obtaining a heatmap based on the plurality of weighted standardized metrics of the electrophysiological signal for each of a plurality of epochs and/or the sum of the plurality of weighted standardized metrics of the electrophysiological signal for each of the plurality of epochs.

In an embodiment, obtaining a quality score comprises obtaining an average of the sum of the plurality of weighted standardized metrics for each of the plurality of epochs.

In an embodiment (not shown) a computer-implemented method comprising the steps of the method for obtaining a plurality of quality metrics for electrophysiological signals and/or the method for obtaining a quality score percentage function for electrophysiological signals and/or the method for obtaining a quality score for an electrophysiological signal is provided.

In an embodiment (not shown) a computer program product comprising instructions which, when the program is executed by a computer, cause the computer to carry out the steps of the method for obtaining a plurality of quality metrics for electrophysiological signals and/or the method for obtaining a quality score percentage function for electrophysiological signals and/or the method for obtaining a quality score for an electrophysiological signal is provided.

While various embodiments of the present disclosure have been described above, it should be understood that they have been presented by way of example only, and not by way of limitation. Likewise, the various diagrams may depict an example architectural or configuration, which are provided to enable persons of ordinary skill in the art to understand exemplary features and functions of the present disclosure. Such persons would understand, however, that the present disclosure is not restricted to the illustrated example architectures or configurations, but can be implemented using a variety of alternative architectures and configurations. Additionally, as would be understood by persons of ordinary skill in the art, one or more features of one embodiment can be combined with one or more features of another embodiment described herein. Thus, the breadth and scope of the present disclosure should not be limited by any one of the above-described exemplary embodiments.

It is also understood that any reference to an element herein using a designation such as "first," "second," and so forth does not generally limit the quantity or order of those elements. Rather, these designations can be used herein as a convenient means of distinguishing between two or more elements or instances of an element. Thus, a reference to first and second elements does not mean that only two elements can be employed, or that the first element must precede the second element in some manner.

Additionally, a person having ordinary skill in the art would understand that information and signals can be represented using any one of a variety of different technologies and techniques. For example, data, instructions, commands, information, signals, bits and symbols, for example, which may be referenced in the above description can be represented by voltages, currents, electromagnetic waves, magnetic fields or particles, optical fields or particles, or any combination thereof.

A skilled person would further appreciate that any one of the various illustrative logical blocks, units, processors, means, circuits, methods and functions described in connection with the aspects disclosed herein can be implemented by electronic hardware (e.g., a digital implementation, an analog implementation, or a combination of the two), firmware, various forms of program or design code incorporating instructions (which can be referred to herein, for convenience, as "software" or a "software unit"), or any combination of these techniques.

To clearly illustrate this interchangeability of hardware, firmware and software, various illustrative components, blocks, units, circuits, and steps have been described above generally in terms of their functionality.

Whether such functionality is implemented as hardware, firmware or software, or a combination of these techniques, depends upon the particular application and design constraints imposed on the overall system. Skilled artisans can implement the described functionality in various ways for each particular application, but such implementation decisions do not cause a departure from the scope of the present disclosure. In accordance with various embodiments, a processor, device, component, circuit, structure, machine, unit, etc. can be configured to perform one or more of the functions described herein. The term "configured to" or "configured for" as used herein with respect to a specified operation or function refers to a processor, device, component, circuit, structure, machine, unit, etc. that is physically constructed, programmed and/or arranged to perform the specified operation or function.

Furthermore, a skilled person would understand that various illustrative logical blocks, units, devices, components and circuits described herein can be implemented within or performed by an integrated circuit (IC) that can include a general purpose processor, a digital signal processor (DSP), an application specific integrated circuit (ASIC), a field programmable gate array (FPGA) or other programmable logic device, or any combination thereof. The logical blocks, units, and circuits can further include antennas and/or transceivers to communicate with various components within the device. A general purpose processor can be a microprocessor, but in the alternative, the processor can be any conventional processor, controller, or state machine. A processor can also be implemented as a combination of computing devices, e.g., a combination of a DSP and a microprocessor, a plurality of microprocessors, one or more microprocessors in conjunction with a DSP core, or any other suitable configuration to perform the functions described herein. If implemented in software, the functions can be stored as one or more instructions or code on a computer-readable medium. Thus, the steps of a method or algorithm disclosed herein can be implemented as software stored on a computer-readable medium.

Computer-readable media includes both computer storage media and communication media including any medium that can be enabled to transfer a computer program or code from one place to another. A storage media can be any available media that can be accessed by a computer. By way of example, and not limitation, such computer-readable media can include RAM, ROM, EEPROM, CD-ROM or other optical disk storage, magnetic disk storage or other magnetic storage devices, or any other medium that can be used to store desired program code in the form of instructions or data structures and that can be accessed by a computer.

In this document, the term "unit" as used herein, refers to software, firmware, hardware, and any combination of these elements for performing the associated functions described herein. Additionally, for purpose of discussion, the various units are described as discrete units; however, as would be apparent to one of ordinary skill in the art, two or more units may be combined to form a single unit that performs the associated functions according to embodiments of the present disclosure.

Additionally, memory or other storage, as well as communication components, may be employed in embodiments of the present disclosure. It will be appreciated that, for clarity purposes, the above description has described embodiments of the present disclosure with reference to different functional units and processors. However, it will be apparent that any suitable distribution of functionality between different functional units, processing logic elements or domains may be used without detracting from the present disclosure. For example, functionality illustrated to be performed by separate processing logic elements, or controllers, may be performed by the same processing logic element, or controller. Hence, references to specific functional units are only references to a suitable means for providing the described functionality, rather than indicative of a strict logical or physical structure or organization.

Various modifications to the implementations described in this disclosure will be readily apparent to those skilled in the art, and the general principles defined herein can be applied to other implementations without departing from the scope of the claims. Thus, the disclosure is not intended to be limited to the implementations shown herein, but is to be accorded the widest scope consistent with the novel features and principles disclosed herein, as recited in the claims below.

## Claims

1. A method for obtaining a quality score for an electrophysiological signal (10), the method including the following steps:

    obtaining a plurality of metrics from the electrophysiological signal (10);
    obtaining a plurality of standardized metrics of the plurality of metrics based on mean and standard deviation values of a plurality of respective metrics from a first predetermined dataset (20) of electrophysiological signals;
    obtaining a weighting parameter for each of the plurality of standardized metrics based on an overlap between respective standardized metrics of the first predetermined dataset (20) of electrophysiological signals and a second predetermined dataset (21) of electrophysiological signals, an overlap between respective standardized metrics of the first predetermined dataset (20) of electrophysiological signals and a third predetermined dataset (22) of electrophysiological signals, and an area of the respective standardized metrics of the first predetermined dataset (20) of electrophysiological signals;

obtaining a plurality of weighted standardized metrics of the plurality of metrics from the electrophysiological signal (10) based on the standardized metrics and the respective weighting parameter; and
obtaining a quality score based on the plurality of weighted standardized metrics of the electrophysiological signal (10).

2. The method according to claim 1, wherein obtaining a quality score comprises obtaining an average of the plurality of weighted standardized metrics of the electrophysiological signal (10).

3. The method according to claim 1 or 2, wherein obtaining a quality score comprises obtaining a plurality of weighted standardized metrics of the electrophysiological signal for each of a plurality of epochs (11), and preferably wherein obtaining a quality score comprises obtaining a sum of the plurality of weighted standardized metrics of the electrophysiological signal for each of the plurality of epochs (11).

4. The method according to claim 3, further comprising obtaining a heatmap based on the plurality of weighted standardized metrics of the electrophysiological signal (10) for each of a plurality of epochs (11) and/or the sum of the plurality of weighted standardized metrics of the electrophysiological signal (10) for each of the plurality of epochs (11).

5. The method according to claim 3 or 4, wherein obtaining a quality score comprises obtaining an average of the sum of the plurality of weighted standardized metrics for each of the plurality of epochs (11).

6. The method according to any one of claims 1 to 5, wherein the plurality of metrics correspond to a plurality of quality metrics obtained by a method for obtaining a plurality of quality metrics for electrophysiological signals (10), the method including the following steps:

obtaining a plurality of metrics from each of:

the first predetermined dataset (20) of electrophysiological signals,
the second predetermined dataset (21) of electrophysiological signals, and
the third predetermined dataset (22) of electrophysiological signals;

obtaining a plurality of standardized metrics of the plurality of metrics of each of the first, second and third predetermined datasets (20, 21, 22) of electrophysiological signals using mean and standard deviation values of the plurality of metrics from the first predetermined dataset (20) of electrophysiological signals; and
obtaining a plurality of quality metrics as a reduced set of metrics from the plurality of standardized metrics based on an overlap between a) the respective standardized metrics of the first predetermined dataset (20) of electrophysiological signals and the second predetermined dataset (21) of electrophysiological signals, and b) the respective standardized metrics of the first predetermined dataset (20) of electrophysiological signals and the third predetermined dataset (22) of electrophysiological signals.

7. The method according to claims 6, wherein obtaining a plurality of quality metrics as a reduced set of metrics comprises:

selecting a number of metrics with the largest overlap between the respective standardized metric of the first predetermined dataset (20) of electrophysiological signals and the second predetermined dataset (21) of electrophysiological signals, and/or
selecting a number of metrics with the smallest overlap between the respective standardized metric of the first predetermined dataset (20) of electrophysiological signals and the third predetermined dataset (22) of electrophysiological signals.

8. The method according to any one of claims 1 to 7, wherein obtaining the plurality of metrics comprises obtaining the plurality of metrics for each of a plurality of epochs (11) and wherein preferably the plurality of metrics from each of the electrophysiological signal and the first, second and third predetermined datasets (20, 21, 22) of electrophysiological signals are extracted from epochs comprising at least 250 samples, preferably between 250 and 7500 samples; and preferably wherein the epochs (11) overlap each other by a predetermined amount, wherein preferably a current evaluated epoch comprises 20% to 100% of new data compared to a previous epoch.

9. The method according to any one of claims 1 to 8, wherein the first predetermined dataset (20) of electrophysiological signals corresponds to data from a gold standard database, preferably obtained by clinical gold standard electrode placement, and more preferably the first predetermined dataset (20) of electrophysiological signals corresponds to full scalp data measured at channel T4, preferably good quality full scalp data.

10. The method according to any one of claims 1 to 9, wherein obtaining a plurality of standardized metrics comprises obtaining a Z-score Z for each of the plurality of metrics, wherein $Z$ is obtained by $Z = \frac{X - \mu}{\sigma}$, wherein X is the respective metric, $\mu$ is the mean value of the respective metric of the first predetermined dataset (20) of electrophysiological signals and $\sigma$ is the standard deviation value of the respective metric of the first predetermined dataset (20) of electrophysiological signals.

11. The method according to any one of claims 1 to 10, wherein obtaining a quality score comprises applying a quality score percentage function.

12. The method according to any one of claims 1 to 11, wherein the weighting parameter $W$ is obtained by

$$W = \left(\frac{A-B}{C}\right)^2$$

, wherein $A$ is the overlap between the respective standardized metrics of the first predetermined dataset (20) of electrophysiological signals and the second predetermined dataset (21) of electrophysiological signals, $B$ is the overlap between the respective standardized metrics of the first predetermined dataset (20) of electrophysiological signals and the third predetermined dataset (22) of electrophysiological signals, and $C$ is the area of the respective standardized metrics of the first predetermined dataset (20) of electrophysiological signals.

13. The method according to any one of claims 1 to 12, wherein the plurality of weighted standardized metrics are obtained by multiplying the plurality of standardized metrics with the respective weighting parameter.

14. A computer-implemented method comprising the steps of the method according to any one of claims 1 to 13.

15. A computer program product comprising instructions which, when the program is executed by a computer, cause the computer to carry out the steps of the method according to any one of claims 1 to 13.

$$Z = \frac{X - \mu}{\sigma}$$

FIG. 1

FIG. 2

$$Z = \frac{X - \mu}{\sigma}(W)$$

| | Epoch 1 | Epoch 2 | Epoch 3 | Epoch N |
|---|---|---|---|---|
| Metric 1 | W1*Z1 | W1*Z1 | W1*Z1 | W1*Z1 |
| Metric N | WN*ZN | WN*ZN | WN*ZN | WN*ZN |
| | Sum(W1*Z1 - WN*ZN) | Sum(W1*Z1 - WN*ZN) | Sum(W1*Z1 - WN*ZN) | Sum(W1*Z1 - WN*ZN) |

FIG. 3

FIG. 4

## Quality index to percentage score

**FIG. 5**

---

601
obtaining a plurality of metrics from each of: a first predetermined dataset of electrophysiological signals, a second predetermined dataset of electrophysiological signals, and a third predetermined dataset of electrophysiological signals

602
obtaining a plurality of standardized metrics of the plurality of metrics of each of the first, second and third predetermined datasets of electrophysiological signals using mean and standard deviation values of the plurality of metrics from the first predetermined dataset of electrophysiological signals

603
obtaining a plurality of quality metrics as a reduced set of metrics from the plurality of standardized metrics based on an overlap between a) the respective standardized metrics of the first predetermined dataset of electrophysiological signals and the second predetermined dataset of electrophysiological signals, and b) the respective standardized metrics of the first predetermined dataset of electrophysiological signals and the third predetermined dataset of electrophysiological signals

**FIG. 6**

701

obtaining a plurality of metrics from each of: a first predetermined dataset of electrophysiological signals, a second predetermined dataset of electrophysiological signals, and a third predetermined dataset of electrophysiological signals

702

obtaining a plurality of standardized metrics of the plurality of metrics of each of the first, second and third predetermined datasets of electrophysiological signals using mean and standard deviation values of the plurality of metrics from the first predetermined dataset of electrophysiological signals

703

obtaining a weighing parameter $W$ for each of the plurality of standardized metrics based on an overlap between respective standardized metrics of the first predetermined dataset of electrophysiological signals and the second predetermined dataset of electrophysiological signals, an overlap between respective standardized metrics of the first predetermined dataset of electrophysiological signals and the third predetermined dataset of electrophysiological signals, and an area of the respective standardized metrics of the first predetermined dataset of electrophysiological signals

704

obtaining a plurality of weighted standardized metrics of the first predetermined dataset of electrophysiological signals based on the standardized metrics and the respective weighing parameter

705

obtaining a quality score percentage function based on the plurality of weighted standardized metrics of the first predetermined dataset of electrophysiological signals

FIG. 7

801

obtaining a plurality of metrics from the electrophysiological signal

802

obtaining a plurality of standardized metrics of the plurality of metrics based on mean and standard deviation values of a plurality of respective metrics from a first predetermined dataset of electrophysiological signals

803

obtaining a weighing parameter for each of the plurality of standardized metrics based on an overlap between respective standardized metrics of the first predetermined dataset of electrophysiological signals and a second predetermined dataset of electrophysiological signals, an overlap between respective standardized metrics of the first predetermined dataset of electrophysiological signals and a third predetermined dataset of electrophysiological signals, and an area of the respective standardized metrics of the first predetermined dataset of electrophysiological signals

804

obtaining a plurality of weighted standardized metrics of the plurality of metrics from the electrophysiological signal based on the standardized metrics and the respective weighing parameter

805

obtaining a quality score based on the plurality of weighted standardized metrics of the electrophysiological signal

FIG. 8

Europäisches
Patentamt

European
Patent Office

Office européen
des brevets

## EUROPEAN SEARCH REPORT

Application Number

EP 22 20 6530

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 335 630 A1 (MENSIA TECH [FR]) 20 June 2018 (2018-06-20) * abstract * * paragraphs [0017], [0050], [0083], [0086]; claims 1-4 * * the whole document * & WO 2018/109166 A1 (MENSIA TECH [FR]) 21 June 2018 (2018-06-21) | 1-15 | INV. A61B5/369 A61B5/00 |
| A | BARTHELEMY QUENTIN ET AL: "The Riemannian Potato Field: A Tool for Online Signal Quality Index of EEG", IEEE TRANSACTIONS ON NEURAL SYSTEMS AND REHABILITATION ENGINEERING, IEEE, USA, vol. 27, no. 2, 1 February 2019 (2019-02-01), pages 244-255, XP011710079, ISSN: 1534-4320, DOI: 10.1109/TNSRE.2019.2893113 [retrieved on 2019-02-08] * abstract * * sect.II, V * * figures 1,2 * * the whole document * | 1-15 | |

TECHNICAL FIELDS SEARCHED (IPC)

A61B

The present search report has been drawn up for all claims

| Place of search | Date of completion of the search | Examiner |
|---|---|---|
| The Hague | 25 April 2023 | Delval, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT**
**ON EUROPEAN PATENT APPLICATION NO.**

EP 22 20 6530

This annex lists the patent family members relating to the patent documents cited in the above-mentioned European search report.
The members are as contained in the European Patent Office EDP file on
The European Patent Office is in no way liable for these particulars which are merely given for the purpose of information.

25-04-2023

| Patent document cited in search report | | Publication date | Patent family member(s) | | Publication date |
|---|---|---|---|---|---|
| EP 3335630 | A1 | 20-06-2018 | EP | 3335630 A1 | 20-06-2018 |
| | | | EP | 3554361 A1 | 23-10-2019 |
| | | | US | 2021282718 A1 | 16-09-2021 |
| | | | WO | 2018109166 A1 | 21-06-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **NATHAN F. et al.** Characterizing contact impedance, signal quality and robustness as a function of the cardinality and arrangement of fingers on dry contact EEG electrodes. *36th Annual International Conference of the IEEE Engineering in Medicine and Biology Society,* 2014
- **TAUTAN A. M. et al.** Signal Quality in Dry Electrode EEG and the Relation to Skin-electrode Contact Impedance Magnitude. *BIODEVICES2014 -International Conference on Biomedical Electronics and Devices2*
- **FIEDLER P. et al.** Novel flexible Dry multipin electrodes for EEG: Signal quality and interfacial impedance of Ti and TiN coatings. *35th Annual International Conference of the IEEE Engineering in Medicine and Biology Society (EMBC),* 2013
- **LINS CALDAS A. S. et al.** Towards Automatic EEG Signal Denoising by Quality Metric Optimization. *International Joint Conference on Neural Networks (IJCNN),* 2020
- **THEA RADÈNTZ.** Signal Quality Evaluation of Emerging EEG Devices. *Front. Physiol.,* 14 February 2018
- **LIU X.** Enhanced Auditory Steady-State Response Using an Optimized Chirp Stimulus-Evoked Paradigm. *Sensors (Basel),* 12 February 2019, vol. 19 (3), 748
- **URIGÜEN J.A. et al.** *EEG artifact removal-state-of-the-art and guidelines*
- **BARRY R. J.** Characterizing pink and white noise in the human electroencephalogram. *J Neural Eng.,* 16 March 2021, vol. 18 (3
- **FICKLING, S.D et al.** Towards Automatic EEG Signal Denoising by Quality Metric Optimization. *International Joint Conference on Neural Networks (IJCNN),* 2020